# EUROPÄISCHE PATENTSCHRIFT

(11) **EP 3 357 527 B1**
(45) Veröffentlichungstag und Bekanntmachung des Hinweises auf die Patenterteilung: **07.10.2020**
(21) Anmeldenummer: 18153124.5
(22) Anmeldetag: 24.01.2018
(51) Int. Cl.: A61M 5/162, A61J 1/14, A61M 5/14, A61J 1/10

(54) **EINSTECHTEIL FÜR EIN MEDIZINISCHES INFUSIONSSYSTEM, TROPFKAMMER UND INFUSIONSSYSTEM**
SPIKE FOR A MEDICAL INFUSION SYSTEM, DRIP CHAMBER AND INFUSION SYSTEM
ÉLÉMENT PIQUEUR POUR UN SYSTÈME DE PERFUSION MÉDICALE, CHAMBRE DE GOUTTE-À-GOUTTE ET SYSTÈME DE PERFUSION

(30) Priorität: 03.02.2017 DE 102017201755
(43) Veröffentlichungstag der Anmeldung: 08.08.2018
(73) Patentinhaber: B. Braun Melsungen AG, 34212 Melsungen (DE)
(72) Erfinder: SCHLITT, Christof, 34621 Obergrenzebach (DE); LUTZ, Manfred, 34286 Spangenberg (DE)
(74) Vertreter: Patentanwälte Ruff, Wilhelm, Beier, Dauster & Partner mbB

(56) Entgegenhaltungen:
- WO-A2-02/064438
- WO-A2-2010/029371
- DE-A1- 3 901 068
- DE-A1-102007 061 346
- US-A1- 2014 014 210

## Beschreibung

Die Erfindung betrifft ein Einstechteil für ein medizinisches Infusionssystem mit einem Einstechdorn, der von wenigstens einem Kanal durchsetzt ist, und der eine Dornspitze aufweist, deren erster Mantelflächenteil - auf eine Mittellängsachse bezogen - zu einem Stirnende hin verjüngt ist. Die Erfindung betrifft zudem eine Tropfkammer und ein Infusionssystem mit einem entsprechenden Einstechteil.

Ein derartiges Einstechteil für Infusionsgeräte ist aus der Offenlegungsschrift DE 197 48 497 A1 bekannt. Das bekannte Infusionsgerät weist einen Einstechdorn auf, der an eine Tropfkammer angeformt ist. Der Einstechdorn enthält einen Flüssigkeitskanal sowie einen Luftkanal und weist eine spitz zulaufende Dornspitze auf.

Weitere Einstechteile sind aus den Dokumenten WO 2010/029371, DE 10 2007 061346, US 2014/014210, WO 02/064438 und DE 39 01 068 bekannt.

Aufgabe der Erfindung ist es, ein Einstechteil, eine Tropfkammer und ein Infusionssystem der eingangs genannten Art zu schaffen, die ein geringeres Verletzungspotential bergen und dennoch eines relativ geringen Kraftaufwandes bei einem Einstechen eines Flüssigkeitsbehälters bedürfen.

Diese Aufgabe wird für das Einstechteil dadurch gelöst, dass ein gegenüberliegender zweiter Mantelflächenteil eine zu dem Stirnende hin gebrochene, geneigte Schneidfläche aufweist. Die Erfindung bezieht sich insbesondere auf schwerkraftbetreibbare Infusionssysteme. Das Einstechteil weist ein Gehäuse auf, das vorzugsweise als Teil einer Tropfkammer ausgebildet ist. Durch die erfindungsgemäße Lösung lässt sich eine Dornspitze schaffen, welche ein geringeres Verletzungsrisiko darstellt. Zugleich lassen sich Einstechsiegel von Flüssigkeitsbehältern mit relativ geringem Kraftaufwand durchstechen. Als erste und zweite Mantelflächenteile sind Außenkonturen der Dornspitze des Einstechdorns zu verstehen, die einander gegenüberliegen.

In Ausgestaltung der Erfindung sind der verjüngte erste Mantelflächenteil und der gegenüberliegende zweite Mantelflächenteil zu dem Stirnende hin gegenseitig zueinander geneigt. Somit wird vorteilhaft eine Geometrie geschaffen, bei welcher sich das Einstechteil in Richtung der Tropfkammer aufweitet, so dass ein der Tropfkammer entgegengesetzter Abschnitt einen geringeren Querschnitt aufweist. Die Geometrie ist nach Art einer doppelseitigen Schneide gestaltet, da beide Mantelflächenteile gegensinnig zueinander zu einem Stirnende der Dornspitze hin schräg zulaufen.

In weiterer Ausgestaltung der Erfindung ist der erste Mantelflächenteil geometrisch stetig verjüngt. Die Verjüngung weist somit keine Stufen, Absätze oder Kanten auf. Dies ermöglicht ein Einstechen des Einstechteils in ein Einstechsiegel oder einen Verschlussstopfen eines Flüssigkeitsbehälters bei relativ gleichbleibendem Widerstand.

In weiterer Ausgestaltung der Erfindung weist der erste Mantelflächenteil eine gekrümmte oder gewölbte Kontur auf. Die Krümmung oder Wölbung ist vorzugsweise konvex oder ballig ausgeführt.

In weiterer Ausgestaltung der Erfindung ist die Schneidfläche durch eine quer verlaufende Kante gebrochen und zu dem Stirnende hin eben gestaltet. Dadurch ergibt sich eine gleichbleibende Schneidkraft oder Einstechkraft über die Länge der Schneidfläche.

In weiterer Ausgestaltung der Erfindung ist die Schneidfläche relativ zu einer Radialebene der Mittellängsachse des Einstechdorns in einem Winkelbereich zwischen 30° und 70°, bevorzugt zwischen 45° und 60°, geneigt. In diesem Winkelbereich lässt sich vorteilhaft ein gutes Einstechverhalten des Einstechdorns erzielen, so dass der Einstechdorn mit relativ kleinem Kraftaufwand einer Bedienperson ein Einstechsiegel oder einen Verschlussstopfen eines Flüssigkeitsbehälters durchstechen kann.

In weiterer Ausgestaltung der Erfindung geht der zweite Mantelflächenteil - in Längsrichtung gesehen - entgegengesetzt zu dem Stirnende in einen Konturbereich über, in dem ein Flüssigkeitskanal mündet. Der Konturbereich ist vorteilhaft dergestalt ausgelegt, dass in einem Gebrauchszustand des Einstechteils, in welchem das Einstechteil mit dem Flüssigkeitsbehälter verbunden ist, eine Flüssigkeit aus dem Flüssigkeitsbehälter in den Flüssigkeitskanal strömen kann. Der Konturbereich weist eine Mündungsöffnung des Flüssigkeitskanals auf.

In weiterer Ausgestaltung der Erfindung ist in dem ersten Mantelflächenteil eine Öffnung eines Belüftungskanals vorgesehen, die gegenüberliegend zu der Schneidfläche des zweiten Mantelflächenteiles vorgesehen ist. Somit ist die Gefahr einer Durchmischung von in den Flüssigkeitsbehälter hinein strömender Luft und aus dem Flüssigkeitsbehälter heraus strömender Flüssigkeit minimiert. Zudem liegt der Lüftungskanal näher an der Dornspitze des Einstechteils und somit in einem Gebrauchszustand höher als die Eintrittsöffnung des Flüssigkeitskanals, so dass die Gefahr der Durchmischung von Luft und Flüssigkeit weiter minimiert wird.

In weiterer Ausgestaltung der Erfindung ist ein zwischen der quer verlaufenden Kante und dem Konturbereich verlaufender Übergangsbereich als ebene, parallel zu der Mittellängsachse verlaufende Übergangsfläche gestaltet. In diesem Bereich verändert sich der Querschnitt des Einstechteils relativ wenig und eine Kraft zum Einstecken in den Flüssigkeitsbehälter bleibt relativ konstant.

Die Aufgabe wird weiterhin gelöst durch eine Tropfkammer für ein medizinisches Infusionssystem mit einem Einstechteil sowie durch ein medizinisches Infusionssystem mit einer Tropfkammer.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
Fig. 1 zeigt eine Schnittdarstellung eines erfindungsgemäßen Infusionssystems mit einer erfindungsgemäßen Tropfkammer und einem erfindungsgemäßen Einstechteil,
Fig. 2 eine isometrische Ansicht eines Einstechdorns des in Fig. 1 gezeigten Einstechteils,
Fig. 3 eine Seitenansicht des in Fig. 2 gezeigten Einstechdorns und
Fig. 4 eine Vorderansicht des in den Fig. 2 und 3 gezeigten Einstechteils.

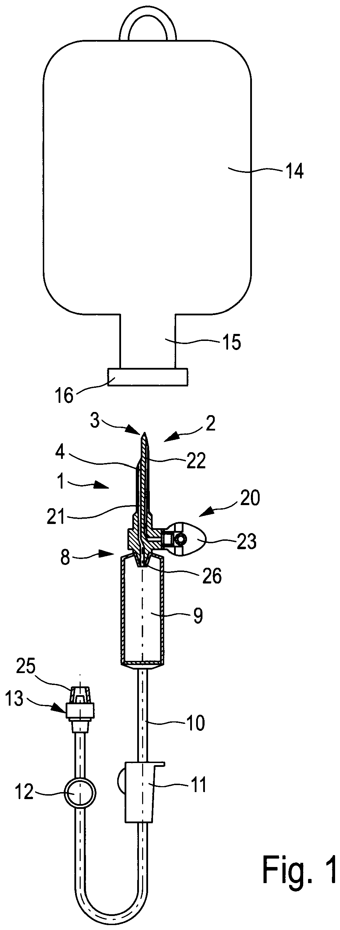

Ein medizinisches Infusionssystem dient der Übertragung einer Flüssigkeit von einem Flüssigkeitsbehälter 14 zu einem Patienten. Die Flüssigkeit wird dabei aus dem Flüssigkeitsbehälter 14 geleitet und über das Infusionssystem zu dem Patienten hin geleitet. Das Infusionssystem weist gemäß Fig. 1 ein Einstechteil 1 auf. In einem in der Betrachtungsebene der Fig. 1 oberen Bereich weist das Einstechteil 1 einen Einstechdorn 2 mit einer Dornspitze 3 auf, die in einer Betrachtungsebene der Fig. 1 nach oben weist, also auf den Flüssigkeitsbehälter 14 zu. Der Flüssigkeitsbehälter 14 ist in einem unteren Bereich, also in einem auf die Dornspitze 3 des Einstechteils 1 zuweisenden Bereich, mit einem Einstechabschnitt 15 versehen, der ein Einstechsiegel 16 in Form eines Verschlussstopfens aufweist. Der Einstechabschnitt 15 ist in einem Auslieferungszustand des Flüssigkeitsbehälters 14 mittels des Einstechsiegels 16 flüssigkeitsdicht verschlossen.

Das Infusionssystem umfasst bei dem dargestellten Ausführungsbeispiel gemäß der Fig. 1 das Einstechteil 1, das ein Gehäuse 8 aufweist, das Teil einer Tropfkammer 9 ist. An der Tropfkammer 9 ist der Einstechdorn 2 einstückig angeformt. Ein Tropfrohr 26, das einstückiger Bestandteil des Einstechteils 1 ist, mündet in die Tropfkammer 9. Der Einstechdorn 2 läuft in die Dornspitze 3 aus. Das Einstechteil 1 weist einen innenliegenden Kanal 4 auf, der hier als Flüssigkeitskanal 21 ausgebildet ist. Weiterhin umfasst das Infusionssystem die Tropfkammer 9, die an den Einstechdorn 2 in einer Durchströmungsrichtung des Infusionssystems stromabwärtig angrenzt, eine Schlauchleitung 10, die stromabwärtig an die Tropfkammer 9 anschließt, welche einen Durchflussregler 11 und einen Zuspritzport 12 aufweist, sowie ein Anschlussstück 13 zum Anschluss an eine Schnittstelle zu dem Patienten, welches mit einer lösbaren Kappe 25 verschlossen ist.

Das dargestellte Infusionssystem ist schwerkraftbetrieben, so dass der Flüssigkeitsbehälter nach unten hängend gehalten ist und der Einstechdorn 3 vertikal von unten her eingestochen wird. Auch die Tropfkammer ist in ihrer Funktionsstellung vertikal ausgerichtet.

In einem Anlieferungszustand des Flüssigkeitsbehälters 14 ist der Einstechabschnitt 15 des Flüssigkeitsbehälter 14 von dem Einstechsiegel 16 verschlossen und muss vor einer Inbetriebnahme des Infusionssystems von der Dornspitze 3 des Einstechteils 1 durchstochen werden, um eine Flüssigkeitsübertragung aus dem Flüssigkeitsbehälter 14 in die Infusionsvorrichtung zu ermöglichen. In einem Anlieferungszustand des Infusionssystems ist das Einstechteil 1 von einer nicht dargestellten Schutzkappe bedeckt, um einer Bedienperson davor zu schützen, sich an der Dornspitze 3 zu verletzten und das Einstechteil 1 vor Verunreinigungen zu schützen.

In der nicht dargestellten Funktionsstellung ist der Flüssigkeitsbehälter 14 mit dem Infusionssystem verbunden. Dabei ist die nicht dargestellte Schutzkappe des Einstechteils 1 entfernt und das Einstechsiegel 16 des Einstechabschnitts 15 von der Dornspitze 3 und dem Einstechdorn 2 des Einstechteils 1 durchdrungen und somit eine Flüssigkeitsübertragung zwischen dem Flüssigkeitsbehälter 14 und der Schlauchleitung 10 sowie dem Patienten ermöglicht.

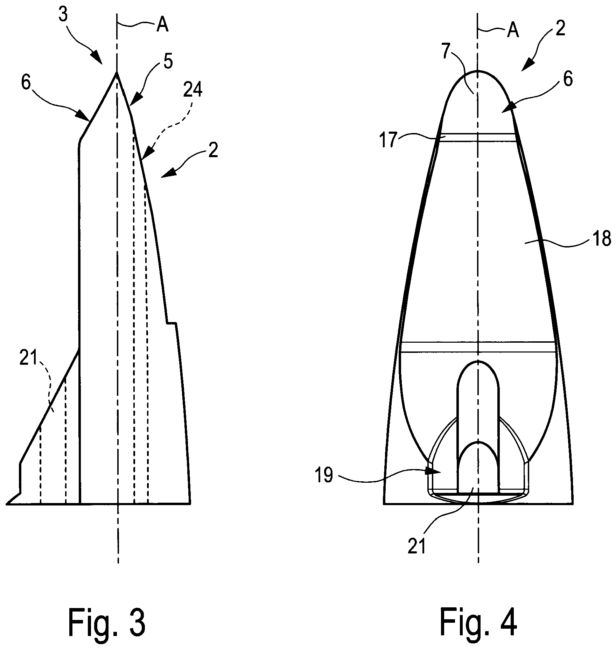

Das Einstechteil 1 weist gemäß Fig. 3 eine Mittellängsachse A auf. In einer Betrachtungsebene der Fig. 3 ist rechts der Mittellängsachse A in einem oberen Bereich ein erster Mantelflächenteil 5 angeordnet. Der erste Mantelflächenteil 5 ist geometrisch stetig verjüngt. Im dargestellten Ausführungsbeispiel beschreibt der erste Mantelflächenteil 5 eine leicht gekrümmte Kontur. Links der Mittellängsachse A, dem ersten Mantelflächenteil 5 gegenüberliegend, ist der zweite Mantelflächenteil 6 angeordnet. Beide Mantelflächenteile 5, 6 sind einander zu einem Stirnende hin, also einem oberen Ende des Einstechdorns 2, gegensinnig zugeneigt. Ein oberer Bereich des ersten Mantelflächenteils 5 bildet mit einem oberen Bereich des zweiten Mantelflächenteils 6 eine Dornspitze 3.

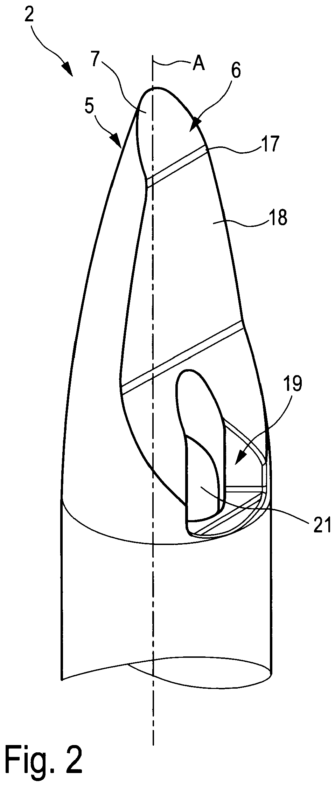

Der zweite Mäntelflächenteil 6 der Dornspitze 3 definiert gemäß Fig. 2 und 4 eine Schneidfläche 7, welche durch eine quer verlaufende Kante 17 gebrochen und zu einem Stirnende des Einstechdorns 2, also einem oberen Ende, hin eben gestaltet ist. Die Schneidfläche 7 ist im dargestellten Ausführungsbeispiel relativ zu einer Radialebene der Mittellängsachse A des Einstechteils 1 um einen Winkel von ca. 45° geneigt.

Der zweite Mantelflächenteil 6 geht in einem unteren Bereich des Einstechteils 1 in einen Konturbereich 19 über, in dem ein Flüssigkeitskanal 21 mündet.

Zwischen der quer verlaufenden Kante 17, die den unteren Abschluss der Schneidfläche 7 bildet, und dem Konturbereich 19 ist gemäß Fig. 2 ein Übergangsbereich 18 als ebene, parallel zu der Mittellängsachse A verlaufende Übergangsfläche gestaltet.

In einem Bereich zwischen dem Einstechdorn 2 und der Tropfkammer 9 ist gemäß Fig. 1 eine Belüftungsvorrichtung 20 angeordnet. Die Belüftungsvorrichtung 20 weist ein außenliegendes Belüftungsventil 23 auf, das einen innenliegenden Belüftungskanal 22 mit der Atmosphäre verbinden kann. Der Flüssigkeitskanal 21 und der Belüftungskanal 22 verlaufen in einem Inneren des Einstechdorns 2 zumindest weitgehend parallel zueinander, wobei der Belüftungskanal 22 in dem Einstechdorn 2 oberhalb des Flüssigkeitskanals 21 mündet, um eine Belüftung des Flüssigkeitsbehälters 14 zu gewährleisten, aber eine Luftbeimischung im Flüssigkeitskanal 21 auszuschließen. Der Belüftungskanal 22 verläuft in einem der Tropfkammer 9 zugewandten Bereich des Einstechdorns 2 rechtwinklig zum Flüssigkeitskanal 21 zu dem Belüftungsventil 23 hin.

Gemäß Fig. 3 weist das Einstechteil 1 an dem ersten Mantelflächenteil 5 eine Öffnung 24 des Belüftungskanals 22 auf, die gegenüberliegend zu dem Flüssigkeitskanal 21 des zweiten Mantelflächenteils 6 angeordnet ist.

Um eine Flüssigkeitsverbindung zwischen dem Flüssigkeitsbehälter 14 und dem Infusionssystem zu schaffen, wird eine nicht dargestellte sterile Verpackung, in welcher das Infusionssystem im Anlieferungszustand verpackt ist, von der Bedienperson geöffnet und das Infusionssystem der Verpackung entnommen. Die Tropfkammer 9 ist in diesem Zustand stromaufwärtig mit dem Einstechteil 1 und stromabwärtig mit der Schlauchleitung 10 verbunden. Die Belüftungsvorrichtung 20 ist seitlich an der Einstechvorrichtung 1 befestigt. In einem Abstand zur Tropfkammer 9 umgibt der Durchflussregler 11 einen Bereich der Schlauchleitung 10. Zu dem Durchflussregler stromabwärtig beabstandet ist der Zuspritzport 12 angeordnet. Ein solcher Zuspritzport 12 ist bei anderen Ausführungsformen des Infusionssystems nicht vorhanden. Stromabwärtig des Zuspritzports 12 ist das Anschlussstück 13 an der Schlauchleitung 10 angebracht und von der lösbaren Kappe verschlossen.

Die nicht dargestellte Schutzkappe des Einstechdorns 2 ist lösbar mit dem Einstechteil 1 verbunden und bedeckt den Einstechdorn 3. Die nicht dargestellte Schutzkappe wird von der Bedienperson entfernt und die Dornspitze 3 des Einstechteils 1 wird in das Einstechsiegel 16 des Einstechabschnitts 15 des Flüssigkeitsbehälters 14 eingestochen. Die von den oberen Abschnitten des ersten und des zweiten Mantelflächenteils 5, 6 gebildete Schneidfläche 7 schneidet dabei in das Einstechsiegel 16 ein. Der an die Dornspitze 3 angrenzende Übergangsbereich 18 wird anschließend durch das Einstechsiegel 16 hindurchgeschoben und ein unterer Bereich des Einstechteils 1 wird durch das Einstechsiegel 16 geschoben. Somit ist eine Flüssigkeitsverbindung zwischen dem Flüssigkeitsbehälter 14 und dem Infusionssystem hergestellt und die Flüssigkeit kann schwerkraftbedingt aus dem Flüssigkeitsbehälter 14 über den Konturbereich 19 durch eine Einmündung des Flüssigkeitskanals 21 in die Tropfkammer 9 strömen. Die Tropfkammer 9 ermöglicht eine Beobachtung des Tropfenfalls und ist zu diesem Zweck aus einem transparenten Material gebildet. Die Tropfkammer 9 weist das innenliegende Tropfrohr 26 auf, das in die Tropfkammer 9 hineinragt, und durch welches die Flüssigkeit in die Tropfkammer 9 eintritt. Der innere Querschnitt des Tropfrohrs 26 stellt eine Verlängerung des Flüssigkeitskanals 21 dar. Durch die Tropfkammer 9 passiert die Flüssigkeit die Schlauchleitung 10 und gelangt über das Anschlussstück 13 zu dem Patienten. Die Schlauchleitung 10 ist aus einem flexiblen und transparenten Material gebildet, um der Bedienperson eine Erkennung von durchströmender Flüssigkeit oder Luft zu ermöglichen. Das Anschlussstück 13 weist einen Außenkegel zur Verbindung mit der Schnittstelle zum Patienten auf. Um bei ausströmender Flüssigkeit einen Druckausgleich im Flüssigkeitsbehälter 14 zu ermöglichen, wird der Flüssigkeitsbehälter 14 während einer Anwendung des Infusionssystems über die Belüftungsvorrichtung 20 belüftet. Das Belüftungsventil 23 öffnet sich zu diesem Zweck, so dass Luft über den Belüftungskanal 22 in den Flüssigkeitsbehälter 14 einströmen kann. Um Verunreinigungen der Flüssigkeit zu vermeiden, weist die Belüftungsvorrichtung 20 einen nicht dargestellten Luftfilter auf. Die Durchströmungsgeschwindigkeit kann dazu mittels des Durchflussreglers 11 variiert werden. Dazu wird mit dem Durchflussregler 11 insbesondere in Form einer Rollenklemme ein Querschnitt des Schlauchs 10 verändert, so dass ein Durchflussvolumen der Flüssigkeit verringert wird. Zudem kann der Querschnitt der Schlauchleitung 10 so weit verringert werden, dass eine Durchströmung der Schlauchleitung 10 vollständig unterbunden wird. Über den Zuspritzport 12 können der Flüssigkeit aus dem Flüssigkeitsbehälter 14 im Bedarfsfall weitere Flüssigkeiten beigemischt werden. Der Zuspritzport 12 ist dazu selbst verschließend ausgebildet.

## Patentansprüche

1. Einstechteil (1) für ein medizinisches Infusionssystem mit einem Einstechdorn (2), der von wenigstens einem Kanal (4) durchsetzt ist, und der eine Dornspitze (3) aufweist, deren erster Mantelflächenteil (5) - auf eine Mittellängsachse (A) bezogen - zu einem Stirnende hin verjüngt ist, wobei ein gegenüberliegender zweiter Mantelflächenteil (6) eine zu dem Stirnende hin gebrochene, geneigte Schneidfläche (7) aufweist, **dadurch gekennzeichnet, dass** der verjüngte erste Mantelflächenteil (5) und der gegenüberliegende zweite Mantelflächenteil (6) zu dem Stirnende hin gegensinnig zueinander geneigt sind, und dass der erste Mantelflächenteil (5) geometrisch stetig verjüngt ist.

2. Einstechteil nach Anspruch 1, **dadurch gekennzeichnet, dass** der erste Mantelflächenteil (5) eine gekrümmte oder gewölbte Kontur aufweist.

3. Einstechteil nach Anspruch 1, **dadurch gekennzeichnet, dass** die Schneidfläche (7) durch eine relativ zu der Mittellängsachse (A) quer verlaufende Kante (17) gebrochen und zu dem Stirnende hin eben gestaltet ist.

4. Einstechteil nach Anspruch 3, **dadurch gekennzeichnet, dass** die Schneidfläche (7) relativ zu einer Radialebene der Mittellängsachse (A) des Einstechdorns (2) in einem Winkelbereich zwischen 30° und 70°, bevorzugt zwischen 45° und 60°, geneigt ist.

5. Einstechteil nach einem der vorhergehenden Ansprüche, **dadurch gekennzeichnet, dass** der zweite Mantelflächenteil (6) - in Längsrichtung gesehen - entgegengesetzt zu dem Stirnende in einen Konturbereich (19) übergeht, in dem ein Flüssigkeitskanal (21) mündet.

6. Einstechteil nach einem der vorhergehenden Ansprüche, **dadurch gekennzeichnet, dass** in dem ersten Mantelflächenteil (5) eine Öffnung (24) eines Belüftungskanals (22) vorgesehen ist, die gegenüberliegend zu der Schneidfläche (7) des zweiten Mantelflächenteiles (6) vorgesehen ist.

7. Einstechteil nach Anspruch 5 oder 6, **dadurch gekennzeichnet, dass** ein zwischen der quer verlaufenden Kante (17) und dem Konturbereich (19) verlaufender Übergangsbereich (18) als ebene, parallel zu der Mittellängsachse (A) verlaufende Übergangsfläche gestaltet ist.

8. Tropfkammer (9) mit einem Einstechteil (1) für ein medizinisches Infusionssystem, wobei das Einstechteil (1) nach einem der vorhergehenden Ansprüche gestaltet ist.

9. Medizinisches Infusionssystem mit einer Tropfkammer (9) nach Anspruch 8 oder mit einem Einstechteil (1) nach einem der Ansprüche 1 bis 7.

## Claims

1. Piercing part (1) for a medical infusion system, with a piercing mandrel (2) through which at least one channel (4) extends and which has a mandrel tip (3) whose first jacket surface part (5) is tapered, relative to a central longitudinal axis A, towards a front end, wherein an opposite second jacket surface part (6) has an inclined cutting face (7) interrupted towards the front end,
**characterized in that**
the tapered first jacket surface part (5) and the opposite second jacket surface part (6) are inclined in mutually opposite directions towards the front end, and **in that** the first jacket surface part (5) is tapered geometrically continuously.

2. Piercing part according to claim 1, **characterized in that** the first jacket surface part (5) has a curved or arched contour.

3. Piercing part according to claim 1, **characterized in that** the cutting face (7) is interrupted by an edge (17) extending transversely relative to the central longitudinal axis (A) and is plane towards the front end.

4. Piercing part according to claim 3, **characterized in that** the cutting face (7) is inclined, relative to a radial plane of the central longitudinal axis (A) of the piercing mandrel (2), in an angle range of between 30° and 70°, preferably of between 45° and 60°.

5. Piercing part according to any of the preceding claims, **characterized in that** the second jacket surface part (6), away from the front end as seen in the longitudinal direction, merges into a contour region (19) in which a liquid channel (21) opens out.

6. Piercing part according to any of the preceding claims, **characterized in that** an opening (24) of a ventilation channel (22) is provided in the first jacket surface part (5), which opening (24) is provided opposite the cutting face (7) of the second jacket surface part (6).

7. Piercing part according to claim 5 or 6, **characterized in that** a transition region (18) extending between the transversely extending edge (17) and the contour region (19) is configured as a plane transition face extending parallel to the central longitudinal axis (A).

8. Drip chamber (9) with a piercing part (1) for a medical infusion system, wherein the piercing part (1) is configured according to any of the preceding claims.

9. Medical infusion system with a drip chamber (9) according to claim 8 or with a piercing part (1) according to any of claims 1 to 7.

## Revendications

1. Partie de piquage (1) pour un système de perfusion médical, comprenant un poinçon de piquage (2) qui est traversé par au moins un canal (4) et qui présente une pointe de poinçon (3) dont la première partie de surface d'enveloppe (5) - rapporté à un axe longitudinal médian (A) - est rétrécie en direction d'une extrémité avant, dans laquelle une seconde partie de surface d'enveloppe (6) opposée présente une surface de coupe (7) inclinée interrompue vers l'extrémité avant, **caractérisée en ce que** la première partie de surface d'enveloppe (5) rétrécie et la seconde partie de surface d'enveloppe (6) opposée sont inclinées dans le sens opposé entre elles en direction de l'extrémité avant et **en ce que** la première partie de surface d'enveloppe (5) est rétrécie géométriquement de manière continue.

2. Partie de piquage selon la revendication 1, **caractérisée en ce que** la première partie de surface d'enveloppe (5) présente une structure coudée ou courbée.

3. Partie de piquage selon la revendication 1, **caractérisée en ce que** la surface de coupe (7) est interrompue par une arête (17) s'étendant transversalement par rapport à l'axe longitudinal médian (A) et est de configuration plane en direction de l'extrémité avant.

4. Partie de piquage selon la revendication 3, **caractérisée en ce que** la surface de coupe (7) est inclinée dans une plage angulaire entre 30° et 70°, de préférence entre 45° et 60°, par rapport à un plan radial de l'axe longitudinal médian (A) du poinçon de piquage (2) .

5. Partie de piquage selon l'une des revendications précédentes, **caractérisée en ce que** la seconde partie de surface d'enveloppe (6) - vu dans le sens longitudinal - se prolonge, de manière opposée à l'extrémité avant, en une zone de contour (19) dans laquelle débouche un canal de liquide (21).

6. Partie de piquage selon l'une des revendications précédentes, **caractérisée en ce que** dans la première partie de surface d'enveloppe (5), une ouverture (24) d'un canal d'aération (22) est prévue, qui est prévue de manière opposée à la surface de coupe (7) de la seconde partie de surface d'enveloppe (6).

7. Partie de piquage selon la revendication 5 ou 6, **caractérisée en ce qu'**une zone de passage (18) passant entre l'arête (17) s'étendant transversalement et la zone de contour (19) est conçue en tant que surface de passage plane s'étendant parallèlement à l'axe longitudinal médian (A).

8. Chambre de goutte-à-goutte (9) comprenant une partie de piquage (1) pour un système de perfusion médical, dans laquelle la partie de piquage (1) est conçue selon l'une des revendications précédentes.

9. Système de perfusion médical comprenant une chambre de goutte-à-goutte (9) selon la revendication 8 ou une partie de piquage (1) selon l'une des revendications 1 à 7.
